# EUROPEAN PATENT APPLICATION

(11) **EP 4 421 075 A1**
(43) Date of publication of application: **28.08.2024**
(21) Application number: 23158761.9
(22) Date of filing: 27.02.2023
(51) Int. Cl.: C07D 471/04, C07D 309/04

(54) **PROCESS FOR THE PREPARATION OF VENETOCLAX AND INTERMEDIATES USED THEREIN**

(71) Applicant: KRKA, d.d., Novo mesto, 8501 Novo mesto (SI)
(72) Inventor: Bergant Simoncic, Ana, 8501 Novo mesto (SI)
(74) Representative: Kutzenberger Wolff & Partner

(57) **Abstract**

The invention relates to a process for the synthesis of Venetoclax. The invention further relates to intermediates that are useful in the synthesis of Venetoclax.

## Description

The invention relates to a process for the synthesis of Venetoclax. The invention further relates to intermediates that are useful in the synthesis of Venetoclax.

Venetoclax, also known in the art as GDC-0199, ABT-199, or RG7601 (CAS 1257044-40-8, ATC L01XX52), is a Bcl-2 inhibitor. It is marketed under the brand name VENCLEXTA^{™} by AbbVie, Inc., and is indicated for the treatment of adults with chronic lymphocytic leukemia (CLL), acute myeloid leukemia (AML) or small lymphocytic lymphoma (SLL).

Venetoclax acts as a BH3-mimetic and binds directly to an anti-apoptotic B-cell lymphoma-2 (bcl-2) protein, displacing a pro-apoptotic protein like BIM, which eventually leads to a programmed cell death of CLL cells. Bcl-2 is present in high amounts in CLL cancer cells, where it helps the cells survive for longer in the body and makes them resistant to cancer medicines.

The chemical formula of Venetoclax is 4-(4-{[2-(4-chlorophenyl)-4,4-dimethylcyclohex-1-en-1-yl]methyl}piperazin-1-yl)-N-({3-nitro-4-[(tetrahydro-2H-pyran-4-ylmethyl)amino]phenyl}sulfonyl)-2-(lH-pyrrolo[2,3- b]pyridin-5-yloxy)benzamide and has a structure as represented below:

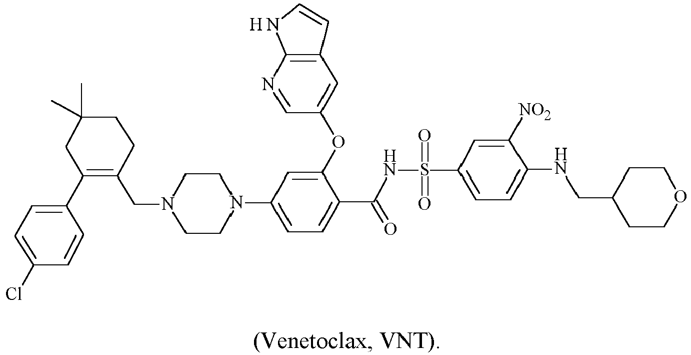

Processes for the preparation of Venetoclax and its intermediates are known in the prior art. In this regard, reference can be made to e.g. EP 2 970 263, IN 2018 41020730, WO 2010 138588, WO 2017 212431, WO 2018 225043, WO 2020 003272, WO 2020 049599, WO 2020 261195, and WO 2021 009770 among others.

The preparation of Venetoclax according to WO 2010 138588 comprises combining 2-((1H-pyrrolo[2,3-b]pyridin-5-yl)oxy)-4-(4-((4'-chloro-5,5-dimethyl-3,4,5,6-tetrahydro-[1,1'-biphenyl]-2-yl)methyl)piperazin-1-yl)benzoic acid with 3-nitro-4-(((tetrahydro-2H-pyran-4-yl)methyl)amino) benzenesulfonamide, wherein 2-((1H-pyrrolo[2,3-b]pyridin-5-yl)oxy)-4-(4-((4'-chloro-5,5-dimethyl-3,4,5,6-tetrahydro-[1,1'-biphenyl]-2-yl)methyl)piperazin-1-yl)benzoic acid is prepared by combining methyl 2-((1H-pyrrolo[2,3-b]pyridin-5-yl)oxy)-4-fluorobenzoate with 1-((4'-chloro-5,5-dimethyl-3,4,5,6-tetrahydro-[1,1'-biphenyl]-2-yl)methyl)piperazine and then converting the product to 2-((lH-pyrrolo[2,3-b]pyridin-5-yl)oxy)-4-(4-((4'-chloro-5,5-dimethyl-3,4,5,6-tetrahydro-[1,1'-biphenyl]-2-yl)methyl)piperazin-1-yl)benzoic acid. Also disclosed is the preparation of 3-nitro-4-(((tetrahydro-2H-pyran-4-yl)methyl)amino) benzenesulfonamide, which is prepared by combining 4-fluoro-3-nitrobenzenesulfonamide with 4-tetrahydropyranmethylamine in the presence of tetrahydrofuran and triethylamine.

The preparation of Venetoclax according to WO 2014 165044 comprises hydrolyzing a compound according to formula (K): 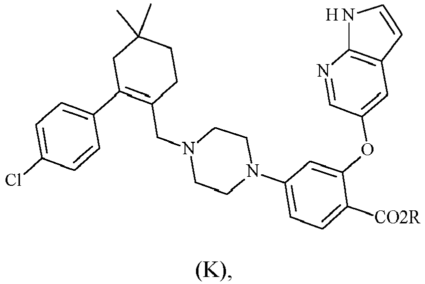 wherein R is C₁ to C₁₂ alkyl, with a tert-butoxide salt, an aprotic organic solvent, and water thereby obtaining compound according to formula (L): 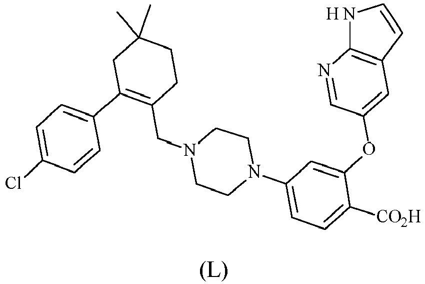 which is then combined with 1-ethyl-3-(3-dimethylaminopropyl) carbodiimide hydrochloride (EDAC), 4-dimethylaminopyridine (DMAP), an organic solvent, and a compound according to formula (N): 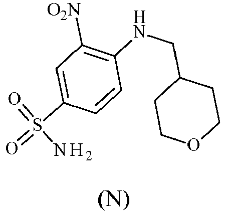 thereby obtaining Venetoclax (VNX).

The compound according to formula (K) is prepared by combining a compound according to formula (d): 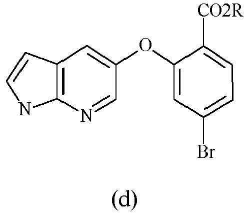 with a compound according to formula (I): 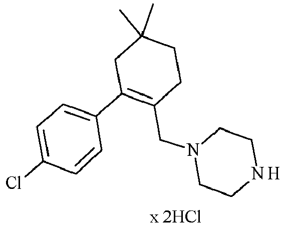 a source of palladium, a tert-butoxide salt, and a phosphine ligand in an aprotic organic solvent.

Ku, Y.-Y. et al. JOC 2019, 84, 4814-4829 and Ku, Yi-Yin; Wendt, Michael D. ACS Symposium Series 2019, 1332, 1-25) already noticed the low regioselectivity and chemoselectivity of SrrAr reactions for the key biarylether building block. With the introduction of the bromide at the 4-position, and differentiating its reactivity from the 2-fluoro position, the regioselectivity issues were diminished. Bromide at the 4-position was also already used in the preparation of compound D of WO2014 165044.

The preparation of Venetoclax according to WO 2020 049599 comprises: (a) treating 1-((4'-chloro-5,5-dimethyl-3,4,5,6-tetrahydro-[1,1'-biphenyl]-2-yl)methyl) piperazine hydrochloride with a base in a solvent thereby obtaining 1-((4'-chloro-5,5-dimethyl-3,4,5,6-tetrahydro-[1,1'-biphenyl]-2-yl)-methyl)piperazine, (b) reacting 1-((4'-chloro-5,5-dimethyl-3,4,5,6-tetrahydro-[1,1'-biphenyl]-2-yl)methyl)piperazine with methyl 2-((1H-pyrrolo[2,3-b]pyridin-5-yl)oxy)-4-fluorobenzoate in the presence of dipotassium hydrogen phosphate in a solvent thereby obtaining 2-((1H-pyrrolo[2,3-b]pyridin-5-yl)oxy)-4-(4-((4'-chloro-5,5-dimethyl-3,4,5,6-tetrahydro-[1,1'-biphenyl]-2-yl)methyl)piperazin-1-yl)benzoate, (c) reacting 2-((lH-pyrrolo[2,3-b]pyridin-5-yl)oxy)-4-(4-((4'-chloro-5,5-dimethyl-3,4,5,6-tetrahydro-[1,1'-biphenyl]-2-yl)methyl)piperazin-1-yl)benzoate in a solvent thereby obtaining 2-((1H-pyrrolo[2,3-b]pyridin-5-yl)oxy)-4-(4-((4'-chloro-5,5-dimethyl-3,4,5,6-tetrahydro-[1,1'-biphenyl]-2-yl)methyl)piperazin-1-yl)benzoic acid, and (d) reacting 2-((1H-pyrrolo[2,3-b]pyridin-5-yl)oxy)-4-(4-((4'-chloro-5,5-dimethyl-3,4,5,6-tetrahydro-[1,1'-biphenyl]-2-yl)methyl)piperazin-1-yl)benzoic acid with 3-nitro-4-(((tetrahydro-2H-pyran-4-yl)methyl)amino)benzenesulfonamide thereby obtaining Venetoclax.

All drug substance preparations contain varying amounts of impurities. These impurities can generally be grouped into categories based on their chemical identity and include "product-related impurities", i.e., impurities that are structurally similar to the active pharmaceutical ingredient (e.g., enantiomers) and "process-related impurities", i.e. impurities introduced by or resulting from the processes used to make the active pharmaceutical ingredient. In the case of the synthesis of Venetoclax, high levels of process-related impurities were observed in the past, after the step of the preparation of a biarylether intermediate, such as e.g. intermediate D.

It is an object of the invention thereby obtaining synthetic routes for the preparation of Venetoclax that have advantages compared to the synthetic routes of the prior art. The synthetic routes should be simple, eco-friendly, cost-effective, robust and well-suited for use on an industrial scale and provide Venetoclax at high yields and high purity. Further, the synthetic routes should start from easily accessible and inexpensive starting materials.

This object has been achieved by the subject-matter of the patent claims.

Surprisingly, it was discovered according to the present invention that the same concept also applies in a novel and bigger molecule comprising more moieties of the final pharmaceutically active ingredient Venetoclax. It is of particular importance to achieve high regioselectivity and chemoselectivity in the last preparation steps of a certain pharmaceutically active compound, as it is more difficult to remove the impurities in the final reaction steps, and as it is also more expensive to do so the closer the intermediate is to the final compound.

According to the present invention, an alternative process for preparing Venetoclax is thus provided, which is simple, eco-friendly, cost-effective, robust and well-suited for use on an industrial scale, as there still remained a need for an alternative process for its preparation in a more cost effective and industrially viable manner.

A first aspect of the invention relates to a process for the synthesis of Venetoclax (VNT) 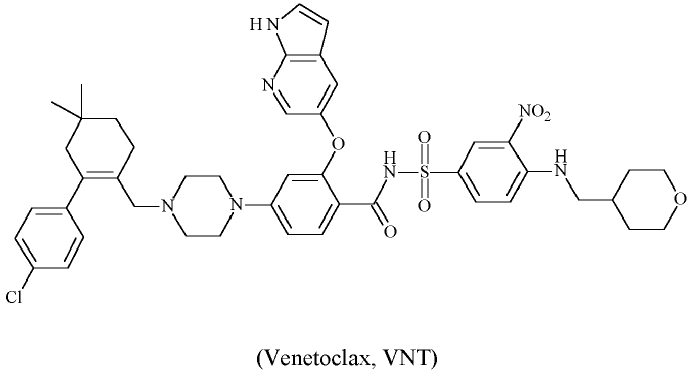 starting from intermediate 4-halo-N-[3-nitro-4-(tetrahydropyran-4-ylmethylamino)phenyl]sulfonyl-2-(1H-pyrrolo[2,3-b]pyridin-5-yloxy)benzamide according to formula (1) 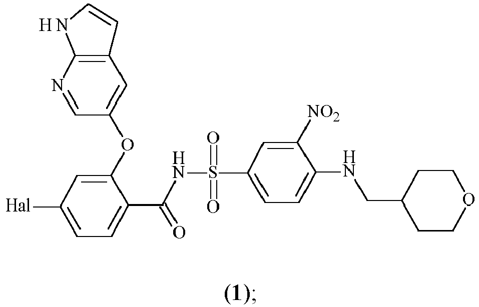
wherein Hal represents -Cl or -Br;
preferably 4-chloro-N-[3-nitro-4-(tetrahydropyran-4-ylmethylamino)phenyl]sulfonyl-2-(1H-pyrrolo [2,3 -b]pyridin-5 -yloxy)benzamide

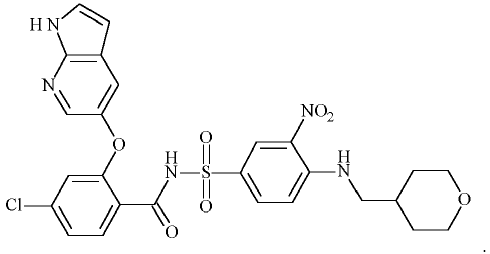

4-Halo-N-[3-nitro-4-(tetrahydropyran-4-ylmethylamino)phenyl]sulfonyl-2-(1H-pyrrolo[2,3-b]pyridin-5-yloxy)benzamide according to formula (1); preferably 4-chloro-N-[3-nitro-4-(tetrahydropyran-4-ylmethylamino)phenyl]sulfonyl-2-(1H-pyrrolo[2,3-b]pyridin-5-yloxy)benzamide; is a key intermediate according to the invention that has been revealed as advantageous building block in the synthesis of Venetoclax (VNT).

The process for the synthesis of Venetoclax (VNT) according to the invention includes two alternatives, in the following referred to as " *Alternative **[f]***" and " *Alternative **[f']***", which both start from the key intermediate 4-halo-N-[3-nitro-4-(tetrahydropyran-4-ylmethylamino)phenyl]sulfonyl-2-(1H-pyrrolo[2,3-b]pyridin-5-yloxy)benzamide according to formula (**1**) (common process step (**e**)).

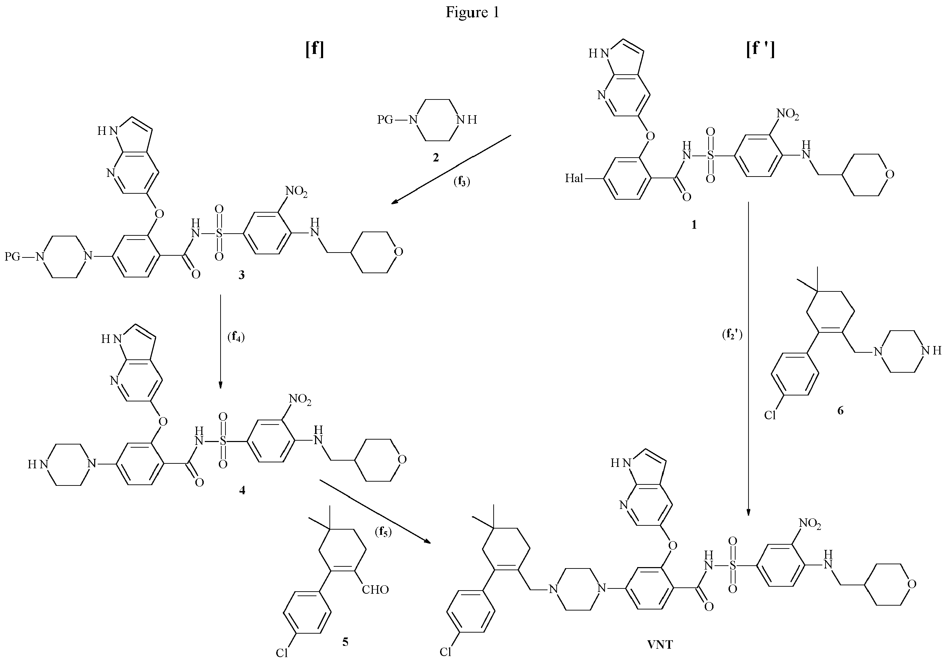

Both alternative synthetic routes are also illustrated in Figure 1.

According to Alternative [f], the process for the synthesis of Venetoclax (VNT) according to the invention comprises the steps of
(e) providing 4-halo-N-[3-nitro-4-(tetrahydropyran-4-ylmethylamino)phenyl]sulfonyl-2-(1H-pyrrolo[2,3-b]pyridin-5-yloxy)benzamide according to formula (1) 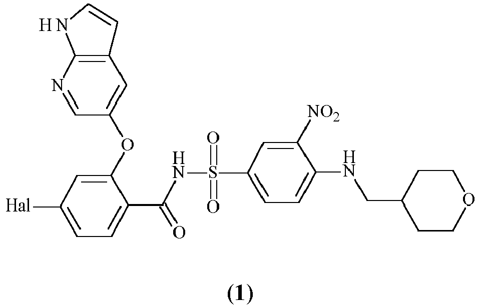
   wherein Hal represents -Cl or -Br;
   preferably 4-chloro-N-[3-nitro-4-(tetrahydropyran-4-ylmethylamino)phenyl]sulfonyl-2-(1H-pyrrolo [2,3 -b]pyridin-5 -yloxy)benzamide 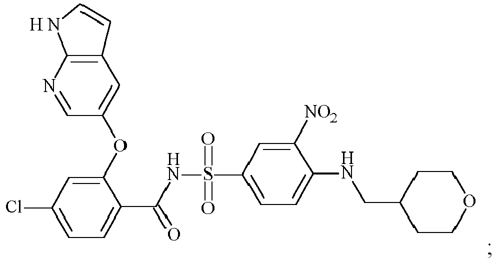
**(f₁)** providing optionally mono-protected piperazine according to formula **(2)** 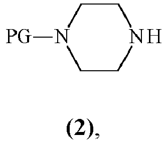 wherein PG represents -H or a protective group;
**(f₂)** providing 2-(4-chlorophenyl)-4,4-dimethyl-cyclohexene-1-carbaldehyde according to formula **(5)** (CAS 1228837-05-5) 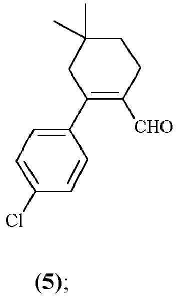 **(f₃)** reacting the 4-halo-N-[3-nitro-4-(tetrahydropyran-4-ylmethylamino)phenyl]sulfonyl-2-(1H-pyrrolo[2,3-b]pyridin-5-yloxy)benzamide according to formula (1); preferably 4-chloro-N-[3-nitro-4-(tetrahydropyran-4-ylmethylamino)phenyl]sulfonyl-2-(1H-pyrrolo[2,3-b]pyridin-5-yloxy)benzamide; with the optionally mono-protected piperazine according to formula (2) thereby obtaining protected N-[3-nitro-4-(tetrahydropyran-4-ylmethylamino)phenyl]sulfonyl-4-piperazin-1-yl-2-(1H-pyrrolo[2,3-b]pyridin-5-yloxy)benzamide according to formula (**3**) (CAS 2134637-30-8) 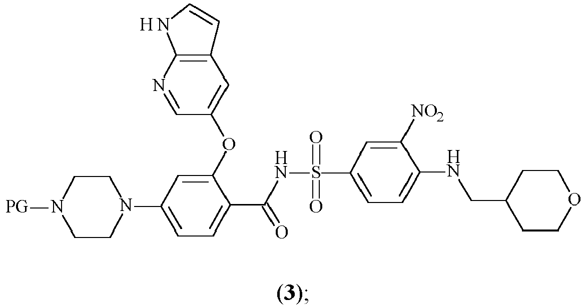
**(f₄)** optionally, when PG does not represent -H, cleaving the protective group PG from the protected N-[3-nitro-4-(tetrahydropyran-4-ylmethylamino)phenyl]sulfonyl-4-piperazin-1-yl-2-(1H-pyrrolo[2,3-b]pyridin-5-yloxy)benzamide according to formula (**3**) thereby obtaining N-[3-nitro-4-(tetrahydropyran-4-ylmethylamino)phenyl]sulfonyl-4-piperazin-1-yl-2-(1H-pyrrolo[2,3-b]pyridin-5-yloxy)benzamide according to formula (**4**) (CAS 2134637-21-9) 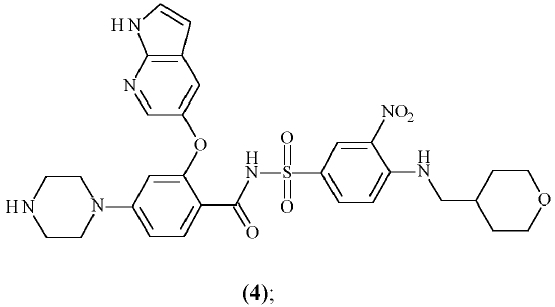 and
**(f₅)** reacting the N-[3-nitro-4-(tetrahydropyran-4-ylmethylamino)phenyl]sulfonyl-4-piperazin-1-yl-2-(1H-pyrrolo[2,3-b]pyridin-5-yloxy)benzamide **(4)** with the 2-(4-chlorophenyl)-4,4-dimethylcyclohexene-1-carbaldehyde according to formula **(5)** in the presence of a reducing agent thereby obtaining the Venetoclax (VNT).

The protective group of the optionally mono-protected piperazine according to formula **(2)** that is provided in step **(f₁)** is preferably tert-butyloxycarbonyl (Boc) such that the mono-protected piperazine according to formula **(2)** has preferably the structure

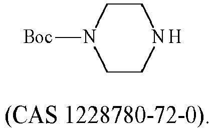

The reaction conditions for step **(f₃)** are not particularly limited. Preferably, the reaction is carried out in the presence of a base. Preferably, the reaction is carried out in the presence of a catalyst, preferably a palladium catalyst.

The reaction conditions for step **(f₄)** are not particularly limited. As the protective group is preferably tert-butyloxycarbonyl (Boc), the protective group is preferably cleaved by means of an acid, e.g. HCl. Preferably, the reaction is carried out in isopropanol (iPrOH). Preferably, the reaction is carried out at a temperature within the range of from 75 to 80°C.

The reaction conditions for step **(f₅)** are not particularly limited. Preferably, the reducing agent is preferably NaBH(OAc)₃. Preferably, the reaction is carried out in toluene and tetrahydrofuran (THF).

The intermediate 2-(4-chlorophenyl)-4,4-dimethyl-cyclohexene-1-carbaldehyde according to formula **(5)** 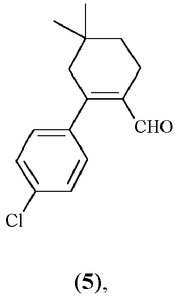 which is provided in step **(f₂)** of the process for the synthesis of Venetoclax (VNT) according to the invention (Alternative **[f]),** is preferably prepared by a process comprising the steps of
**(f₂-i)** providing 3,3-dimethylcyclohexanone according to formula **(11)** (CAS 2979-19-3) 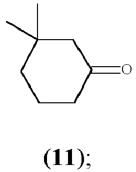
**(f₂-ii)** providing (4-chlorophenyl)boronic acid according to formula **(13)** (CAS 1679-18-1) 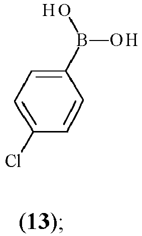
**(f₂-iii)** formylating the 3,3-dimethylcyclohexanone according to formula **(11)** with POCl₃ and dimethylformamide (DMF) (Vilsmeyer reagent) thereby obtaining 2-chloro-4,4-dimethyl-cyclohexene-1-carbaldehyde according to formula **(12)** 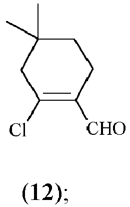 and
**(f₂-iv)** reacting the 2-chloro-4,4-dimethyl-cyclohexene-1-carbaldehyde according to formula **(12)** with the (4-chlorophenyl)boronic acid according to formula **(13)** thereby obtaining the 2-(4-chlorophenyl)-4,4-dimethyl-cyclohexene-1-carbaldehyde according to formula **(5).**

The reaction conditions for step **(f₂-iii)** are not particularly limited. Preferably, the reaction is carried out in toluene. Preferably, the reaction is carried out at a temperature within the range of from 50 to 55°C.

The reaction conditions for step **(f₂-iv)** are not particularly limited. Preferably, the reaction is carried out in the presence of a base, preferably K₂CO₃. Preferably, the reaction is carried out in the presence of a catalyst, preferably Pd(PPh₃)₄. Preferably, the reaction is carried out in acetonitrile (MeCN) and water. Preferably, the reaction is carried out at a temperature within the range of from 75 to 80°C.

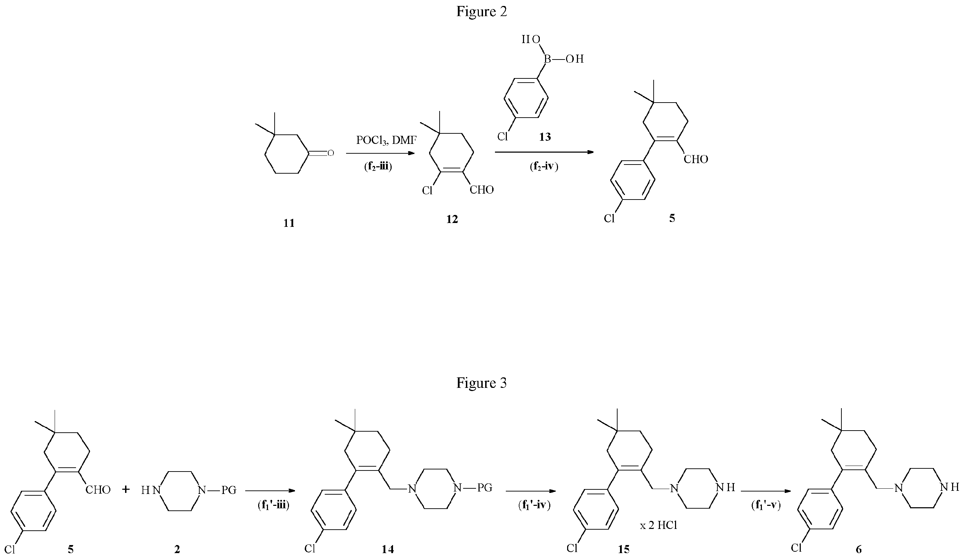

The synthetic route is also illustrated in Figure 2.

According to Alternative **[f** '], the process for the synthesis of Venetoclax (VNT) according to the invention comprises the steps of
(e) providing 4-halo-N-[3-nitro-4-(tetrahydropyran-4-ylmethylamino)phenyl]sulfonyl-2-(1H-pyrrolo[2,3-b]pyridin-5-yloxy)benzamide according to formula (1) 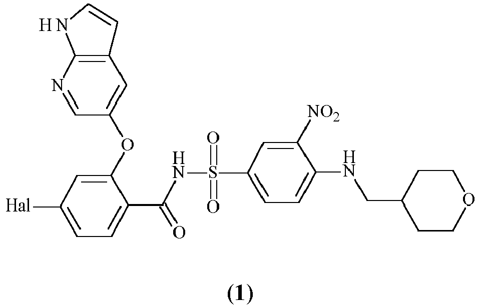
   wherein Hal represents -Cl or -Br;
   preferably 4-chloro-N-[3-nitro-4-(tetrahydropyran-4-ylmethylamino)phenyl]sulfonyl-2-(1H-pyrrolo [2,3 -b]pyridin-5 -yloxy)benzamide 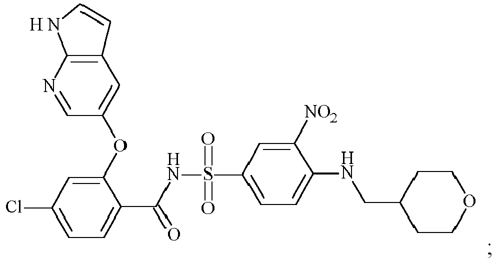
**(f₁')** providing 1-[[2-(4-chlorophenyl)-4,4-dimethyl-cyclohexen-1-yl]methyl]piperazine according to formula **(6)** (CAS 1228780-72-0) 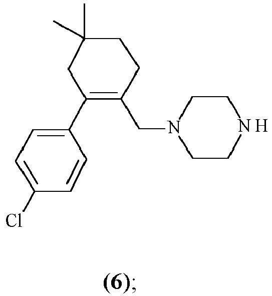 and
**(f₂')** reacting the 4-halo-N-[3-nitro-4-(tetrahydropyran-4-ylmethylamino)phenyl]sulfonyl-2-(1H-pyrrolo[2,3-b]pyridin-5-yloxy)benzamide according to formula **(1);** preferably 4-chloro-N-[3-nitro-4-(tetrahydropyran-4-ylmethylamino)phenyl]sulfonyl-2-(1H-pyrrolo[2,3-b]pyridin-5-yloxy)benzamide; with the 1-[[2-(4-chlorophenyl)-4,4-dimethyl-cyclohexen-1-yl]methyl]piperazine according to formula **(6)** thereby obtaining Venetoclax (VNT).

The reaction conditions for step **(f₂')** are not particularly limited. Preferably, the reaction is carried out in the presence of a base. Preferably, the reaction is carried out in the presence of a catalyst, preferably a palladium catalyst.

The intermediate 1-[[2-(4-chlorophenyl)-4,4-dimethyl-cyclohexen-1-yl]methyl]piperazine according to formula (6) 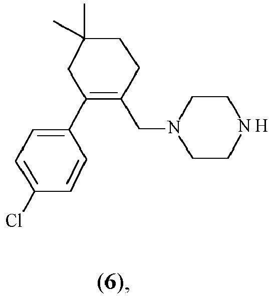 which is provided in step **(f1')** of the process for the synthesis of Venetoclax (VNT) according to the invention (Alternative **[f '])** is preferably prepared by a process comprising the steps of
**(f₁'-i)** providing 2-(4-chlorophenyl)-4,4-dimethyl-cyclohexene-1-carbaldehyde according to formula **(5)** 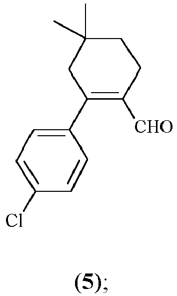
**(f₁'-ii)** providing an optionally mono-protected piperazine according to formula (2) 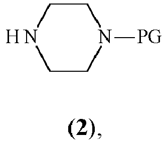 wherein PG represents -H or a protective group;
**(f₁'-iii)** reacting the 2-(4-chlorophenyl)-4,4-dimethyl-cyclohexene-1-carbaldehyde according to formula **(5)** with the optionally mono-protected piperazine according to formula **(2)** in the presence of a reducing agent thereby obtaining protected 1-[[2-(4-chlorophenyl)-4,4-dimethyl-cyclohexen-1-yl]methyl]piperazine according to formula **(14)** 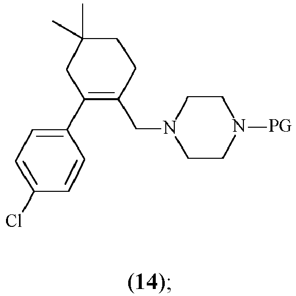
**(f₁'-iv)** optionally, when PG does not represent -H, cleaving the protective group from the protected 1-[[2-(4-chlorophenyl)-4,4-dimethyl-cyclohexen-1-yl]methyl]piperazine according to formula **(14)** thereby obtaining 1-[[2-(4-chlorophenyl)-4,4-dimethyl-cyclohexen-1-yl]methyl]piperazine dihydrochloride according to formula **(15)** 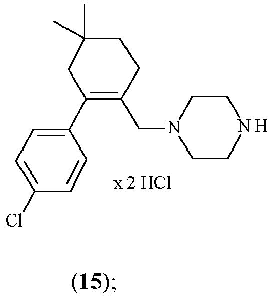 and
**(f₁'-v)** treating the 1-[[2-(4-chlorophenyl)-4,4-dimethyl-cyclohexen-1-yl]methyl]piperazine dihydrochloride according to formula **(15)** with a base thereby obtaining the 1-[[2-(4-chlorophenyl)-4,4-dimethyl-cyclohexen-1-yl]methyl]piperazine according to formula **(6).**

The protective group of the optionally mono-protected piperazine according to formula **(2)** that is provided in step **(f₁'-ii)** is preferably tert-butyloxycarbonyl (Boc) such that the mono-protected piperazine according to formula **(2)** has preferably the structure

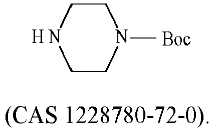

The reaction conditions for step **(f_{1'}-iii)** are not particularly limited. Preferably, the reducing agent is NaBH(OAc)₃. Preferably, the reaction is carried out in toluene and THF.

The reaction conditions for step **(f₁'-iv)** are not particularly limited. As the protective group is preferably tert-butyloxycarbonyl (Boc), the protective group is preferably cleaved by means of an acid, e.g. HCl. Preferably, the reaction is carried out in isopropanol (iPrOH). Preferably, the reaction is carried out at a temperature within the range of from 75 to 80°C.

The reaction conditions for step **(f₁'-v)** are not particularly limited. Preferably, the base is NaOH. Preferably, the reaction is carried out in water and toluene. Preferably, the reaction is carried out at a temperature within the range of from 25 to 30°C.

The synthetic route is also illustrated in Figure 3.

Another aspect of the invention relates to the synthesis of the intermediate 4-halo-N-[3-nitro-4-(tetrahydropyran-4-ylmethylamino)phenyl]sulfonyl-2-(1H-pyrrolo[2,3-b]pyridin-5-yloxy)benzamide according to formula (1); preferably 4-chloro-N-[3-nitro-4-(tetrahydropyran-4-ylmethylamino)phenyl]sulfonyl-2-(1H-pyrrolo[2,3-b]pyridin-5-yloxy)benzamide; which is used as starting material in the process for the synthesis of Venetoclax (VNT) according to the invention (both, Alternative **[f]** and Alternative **[f ']).**

The process for the synthesis of the intermediate 4-halo-N-[3-nitro-4-(tetrahydropyran-4-ylmethylamino)phenyl]sulfonyl-2-(1H-pyrrolo[2,3-b]pyridin-5-yloxy)benzamide according to formula **(1)** according to the invention; preferably 4-chloro-N-[3-nitro-4-(tetrahydropyran-4-ylmethylamino)phenyl]sulfonyl-2-(1H-pyrrolo[2,3-b]pyridin-5-yloxy)benzamide; includes two alternatives, in the following referred to as " *Alternative **[d]***" and " *Alternative **[d']***" which both involve reaction of 4-halo-2-fluoro-benzoic acid according to formula **(7);** preferably 4-chloro-2-fluoro-benzoic acid; 3-nitro-4-(tetrahydropyran-4-ylmethylamino)benzenesulfonamide according to formula **(8),** and 1H-pyrrolo[2,3-b]pyridin-5-ol according to formula **(10)** (common process steps **(a), (b)** and **(c)).**

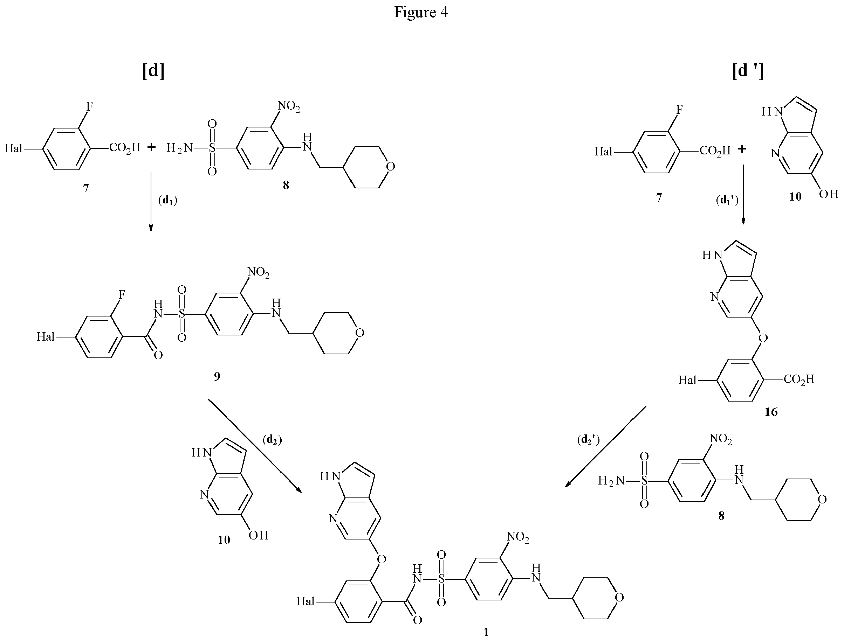

Both alternative synthetic routes are also illustrated in Figure 4.

According to Alternative **[d],** the intermediate 4-halo-N-[3-nitro-4-(tetrahydropyran-4-ylmethylamino)phenyl]sulfonyl-2-(1H-pyrrolo[2,3-b]pyridin-5-yloxy)benzamide according to formula (1) 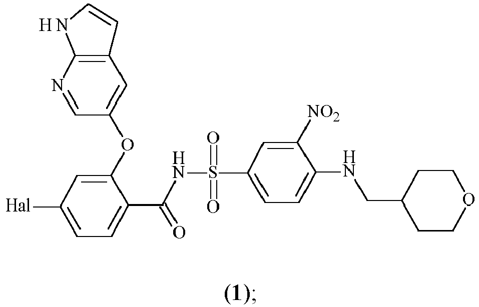
wherein Hal represents -Cl or -Br;
preferably 4-chloro-N-[3-nitro-4-(tetrahydropyran-4-ylmethylamino)phenyl]sulfonyl-2-(1H-pyrrolo [2,3 -b]pyridin-5 -yloxy)benzamide 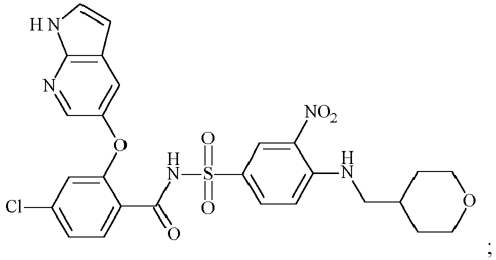
is preferably prepared by a process comprising the steps of
**(a)** providing 4-halo-2-fluoro-benzoic acid according to formula (7) 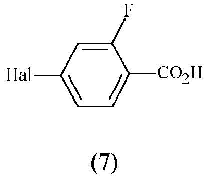
   wherein Hal represents -Cl or -Br;
   preferably 4-chloro-2-fluoro-benzoic acid (CAS 446-30-0);
**(b)** providing 3-nitro-4-(tetrahydropyran-4-ylmethylamino)benzenesulfonamide according to formula (8) 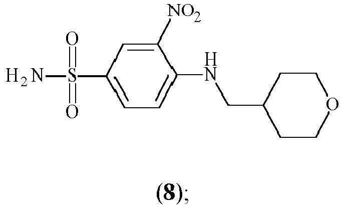
**(c)** providing 1H-pyrrolo[2,3-b]pyridin-5-ol according to formula **(10)** (CAS 98549-88-3) 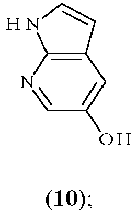
(**d₁**) reacting the 4-halo-2-fluoro-benzoic acid according to formula **(7)** with the 3-nitro-4-(tetrahydropyran-4-ylmethylamino)benzenesulfonamide according to formula **(8)** thereby obtaining 4-halo-2-fluoro-N-[3-nitro-4-(tetrahydropyran-4-ylmethylamino)phenyl]sulfonyl-benzamide according to formula **(9)** 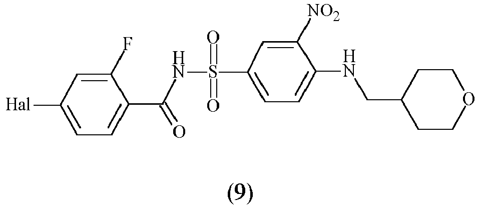
   wherein Hal represents -Br or -Cl;
   preferably 4-chloro-2-fluoro-N-[3-nitro-4-(tetrahydropyran-4-ylmethylamino)phenyl]sulfonyl-benzamide; and
**(d₂)** reacting the 4-halo-2-fluoro-N-[3-nitro-4-(tetrahydropyran-4-ylmethylarnino)phenyl]sulfonyl-benzamide according to formula **(9);** preferably 4-chloro-2-fluoro-N-[3-nitro-4-(tetrahydropyran-4-ylmethylamino)phenyl]sulfonyl-benzamide; with the 1H-pyrrolo[2,3-b]pyridin-5-ol according to formula **(10)** thereby obtaining the 4-halo-N-[3-nitro-4-(tetrahydropyran-4-ylmethylamino)phenyl]sulfonyl-2-(1H-pyrrolo[2,3-b]pyridin-5-yloxy)benzamide according to formula **(1);** preferably 4-chloro-N-[3-nitro-4-(tetrahydropyran-4-ylmethylamino)phenyl]sulfonyl-2-(1H-pyrrolo[2,3-b]pyridin-5-yloxy)benzamide.

The reaction conditions for step (**d₁**) are not particularly limited. Preferably, the reaction is carried out in the presence of a coupling reagent; preferably EDC in combination with DMAP. Preferably, the reaction is carried out in CH₂Cl₂.

The reaction conditions for step **(d₂)** are not particularly limited. Preferably, the reaction is carried out in the presence of a base.

According to Alternative **[d '],** the intermediate the 4-halo-N-[3-nitro-4-(tetrahydropyran-4-ylmethylamino)phenyl]sulfonyl-2-(1H-pyrrolo[2,3-b]pyridin-5-yloxy)benzamide according to formula (1) 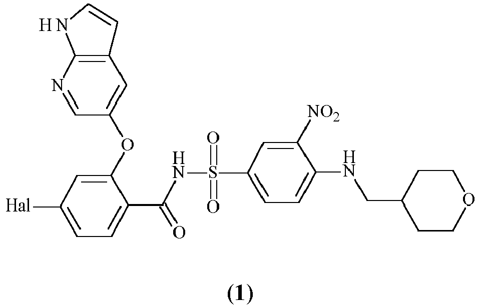
wherein Hal represents -Cl or -Br;
preferably 4-chloro-N-[3-nitro-4-(tetrahydropyran-4-ylmethylamino)phenyl]sulfonyl-2-(1H-pyrrolo [2,3 -b]pyridin-5 -yloxy)benzamide 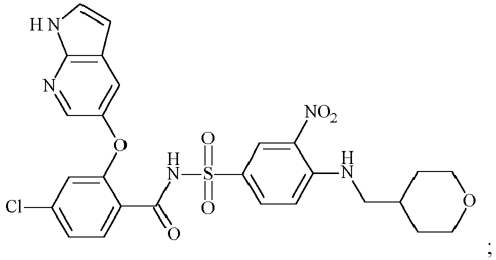
is preferably prepared by a process comprising the steps of
**(a)** providing 4-halo-2-fluoro-benzoic acid according to formula **(7)** 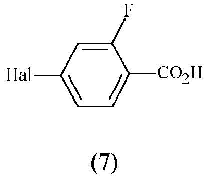
   wherein Hal represents -Cl or -Br;
   preferably chloro-2-fluoro-benzoic acid;
**(b)** providing 3-nitro-4-(tetrahydropyran-4-ylmethylamino)benzenesulfonamide according to formula (8) 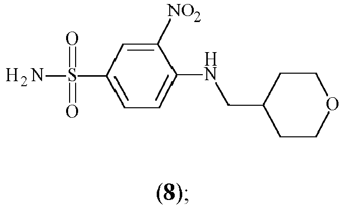
**(c)** providing 1H-pyrrolo[2,3-b]pyridin-5-ol according to formula **(10)** 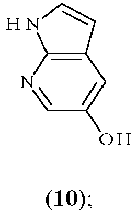
(**d₁'**) reacting the 4-halo-2-fluoro-benzoic acid according to formula **(7);** preferably 4-chloro-2-fluorobenzoic acid; with the 1H-pyrrolo[2,3-b]pyridin-5-ol according to formula **(10)** thereby obtaining 4-halo-2-(1H-pyrrolo[2,3-b]pyridin-5-yloxy)benzoic acid according to formula **(16)** 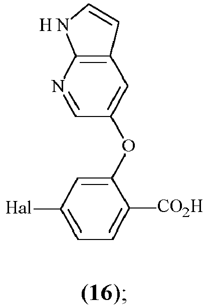
   wherein Hal represents -Cl or -Br;
   preferably 4-chloro-2-(1H-pyrrolo[2,3-b]pyridin-5-yloxy)benzoic acid; and
(**d₂'**) reacting the 4-halo-2-(1H-pyrrolo[2,3-b]pyridin-5-yloxy)benzoic acid according to formula **(16);** preferably 4-chloro-2-(1H-pyrrolo[2,3-b]pyridin-5-yloxy)benzoic acid; with the 3-nitro-4-(tetrahydropyran-4-ylmethylamino)benzenesulfonamide according to formula **(8)** thereby obtaining the 4-halo-N-[3-nitro-4-(tetrahydropyran-4-ylmethylamino)phenyl]sulfonyl-2-(1H-pyrrolo[2,3-b]pyridin-5-yloxy)benzamide according to formula **(1);** preferably 4-chloro-N-[3-nitro-4-(tetrahydropyran-4-ylmethylamino)phenyl]sulfonyl-2-(1H-pyrrolo[2,3-b]pyridin-5-yloxy)benzamide.

The reaction conditions for step **(d₁')** are not particularly limited. Preferably, the reaction is carried out in the presence of a base.

The reaction conditions for step **(d₂')** are not particularly limited. Preferably, the reaction is carried out in the presence of a coupling reagent; preferably EDC in combination with DMAP. Preferably, the reaction is carried out in CH₂Cl₂.

Both, Alternative **[d]** and Alternative **[d ']** start from 4-halo-2-fluoro-benzoic acid according to formula (7); preferably 4-chloro-2-fluoro-benzoic acid; which has two different halogenic substituents (chloro/bromo and fluoro) enabling a better regioselectivity in the substitution reaction.

In WO 2014 165044 a different starting material is used that likewise has two different substituents (bromo and fluoro). Due to the presence of a bromo substituent, however, a palladium catalyst needs to be used in Buchwald-Hartwig amination. In contrast, the present invention does not necessarily involve any starting materials or intermediates that carry bromo substituents. A chloro substituent is preferred. Metal catalysts may therefore be omitted. This is advantageous not only for costs reasons but also in terms of possible contamination of Venetoclax (VNT) with elemental impurities.

Another advantage of the present invention is that 4-halo-2-fluoro-benzoic acid according to formula (7); preferably 4-chloro-2-fluoro-benzoic acid; is used in its acidic form already in the first step of the synthesis, so later on there is no need for hydrolysis of esters or other protective groups. This reduces the overall number of reaction steps.

Additional advantage of the present invention is the early use of 3-nitro-4-(tetrahydropyran-4-ylmethylamino)benzenesulfonamide according to formula (8), by which acyl sulfonamide group is introduced. Its acidic -NH functional group enables different purification methods of intermediates, which are obtained later in the process of the synthesis.

The process for the synthesis of Venetoclax (VNT) according to the invention (Alternative **[f]** or Alternative **[f '])** preferably includes the process for the synthesis of the intermediate the 4-halo-N-[3-nitro-4-(tetrahydropyran-4-ylmethylamino)phenyl]sulfonyl-2-(1H-pyrrolo[2,3-b]pyridin-5-yloxy)-benzamide according to formula **(1)** according to the invention; preferably 4-chloro-N-[3-nitro-4-(tetrahydropyran-4-ylmethylamino)phenyl]sulfonyl-2-(1H-pyrrolo[2,3-b]pyridin-5-yloxy)benzamide (Alternative **[d]** or Alternative **[d ']).** Preferred combinations are:
(i) synthesis according to Alternative **[d]** followed by synthesis according to Alternative **[f];**
(ii) synthesis according to Alternative **[d]** followed by synthesis according to Alternative **[f '];**
(iii) synthesis according to Alternative **[d ']** followed by synthesis according to Alternative **[f]**; or
(iv) synthesis according to Alternative **[d ']** followed by synthesis according to Alternative **[f '].**

Another aspect of the invention relates to intermediate 4-halo-N-[3-nitro-4-(tetrahydropyran-4-ylmethylamino)phenyl]sulfonyl-2-(1H-pyrrolo[2,3-b]pyridin-5-yloxy)benzamide according to formula **(1)** 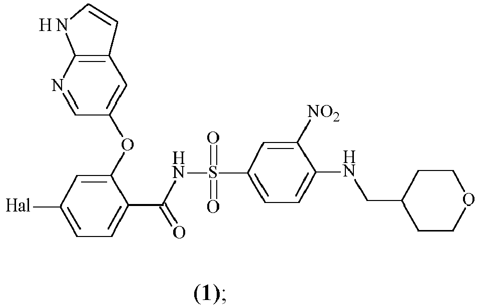
wherein Hal represents -Cl or -Br;
preferably 4-chloro-N-[3-nitro-4-(tetrahydropyran-4-ylmethylamino)phenyl]sulfonyl-2-(1H-pyrrolo [2,3 -b]pyridin-5 -yloxy)benzamide

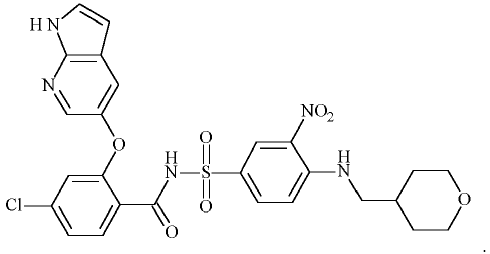

Another aspect of the invention relates to intermediate 4-halo-2-fluoro-N-[3-nitro-4-(tetrahydropyran-4-ylmethylamino)phenyl]sulfonyl-benzamide according to formula (9) 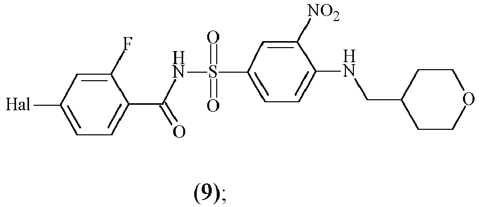
wherein Hal represents -Cl or Br;
preferably 4-chloro-2-fluoro-N-[3-nitro-4-(tetrahydropyran-4-ylmethylamino)phenyl]sulfonyl-benzamide

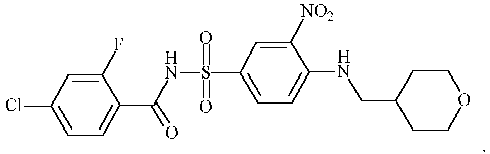

The following examples further illustrate the invention but are not to be construed as limiting its scope.

### Example 1 - synthesis of (9) from (7) and (8) - step (d₁):

A mixture of 2.77 g of 4-chloro-2-fluoro-benzoic acid according to formula (7), 63 mL CH₂Cl₂ and 4.42 mL triethylamine was slowly added to a mixture of 4.00 g of 3-nitro-4-(tetrahydropyran-4-ylmethylamino)benzenesulfonamide according to formula **(8),** 1.94 g of dimethyl aminopyridine (DMAP), 6.912 g of 1-ethyl-3-(3-dimethylaminopropyl)carbodiimide (EDC) and 91 mL CH₂Cl₂ at about 20 - 25°C under inert atmosphere. After about 23 h of stirring at about 20 - 25°C, 72 mL of 9.5 % AcOH (aq.) was added and the phases were separated. The organic phase was washed with 72 mL of 9.5 % AcOH (aq.), followed by 85 mL of 5 % NaHCOs (aq.). The organic phase was evaporated under reduced pressure to obtain 7.308 g of 4-chloro-2-fluoro-N-[3-nitro-4-(tetrahydropyran-4-ylmethylamino)phenyl]sulfonyl-benzamide according to formula (9) (89.27 % chromatographic purity). Further purification afforded (9) with 98.24 % chromatographic purity.

## Claims

1. A process for the synthesis of 4-halo-N-[3-nitro-4-(tetrahydropyran-4-ylmethylamino)phenyl]sulfonyl-2-(1H-pyrrolo[2,3-b]pyridin-5-yloxy)benzamide according to formula **(1)** 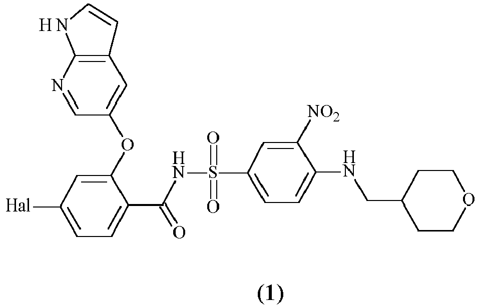
wherein Hal represents -Cl or -Br;
the process comprising the steps of
(a) providing 4-halo-2-fluoro-benzoic acid according to formula (7) 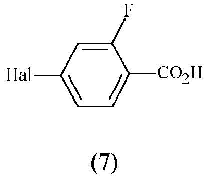 wherein Hal represents -Cl or -Br;
(b) providing 3-nitro-4-(tetrahydropyran-4-ylmethylamino)benzenesulfonamide according to formula **(8)** 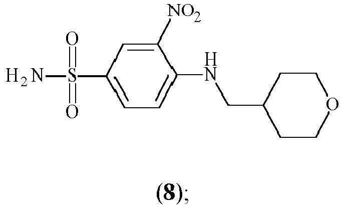
**(c)** providing 1H-pyrrolo[2,3-b]pyridin-5-ol according to formula **(10)** 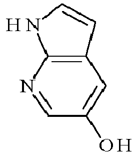 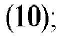 and
**(d)**
(**d₁**) reacting the 4-halo-2-fluoro-benzoic acid according to formula **(7)** with the 3-nitro-4-(tetrahydropyran-4-ylmethylamino)benzenesulfonamide according to formula **(8)** thereby obtaining 4-halo-2-fluoro-N-[3-nitro-4-(tetrahydropyran-4-ylmethylamino)phenyl]sulfonyl-benzamide according to formula **(9)** 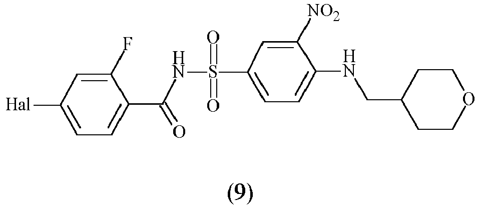 wherein Hal represents -Cl or -Br; and
**(d₂)** reacting the 4-halo-2-fluoro-N-[3-nitro-4-(tetrahydropyran-4-ylmethylamino)phenyl]sulfonyl-benzamide according to formula **(9)** with the 1H-pyrrolo[2,3-b]pyridin-5-ol according to formula **(10);**
or
**(d')**
(**d₁'**) reacting the 4-halo-2-fluoro-benzoic acid according to formula (7) with the 1H-pyrrolo[2,3-b]pyridin-5-ol according to formula **(10)** thereby obtaining 4-halo-2-(1H-pyrrolo[2,3-b]pyridin-5-yloxy)benzoic acid according to formula **(16)** 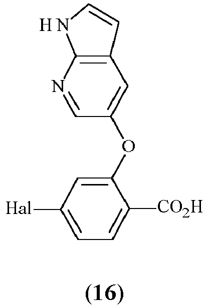 wherein Hal represents -Cl or -Br; and
(**d₂'**) reacting the 4-halo-2-(1H-pyrrolo[2,3-b]pyridin-5-yloxy)benzoic acid according to formula **(16)** with the 3-nitro-4-(tetrahydropyran-4-ylmethylamino)benzenesulfonamide according to formula **(8);**
thereby obtaining the 4-halo-N-[3-nitro-4-(tetrahydropyran-4-ylmethylamino)phenyl]sulfonyl-2-(1H-pyrrolo[2,3-b]pyridin-5-yloxy)benzamide according to formula **(1).**

2. The process according to claim 1, wherein step **(d₁)** or step **(d₂')** is carried out in the presence of a coupling reagent; preferably EDC in combination with DMAP; preferably in CH₂Cl₂.

3. The process according to claim 1 or 2, wherein step **(d₂)** or step (**d₁'**) is carried out in the presence of a base.

4. A process for the synthesis of Venetoclax (VNT) 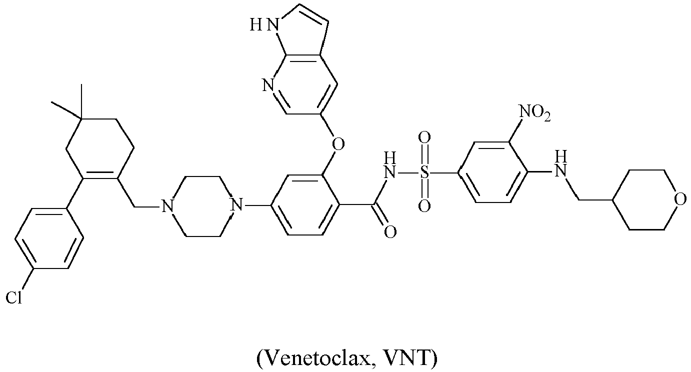 the process comprising the steps of
(e) providing 4-halo-N-[3-nitro-4-(tetrahydropyran-4-ylmethylamino)phenyl]sulfonyl-2-(1H-pyrrolo[2,3-b]pyridin-5-yloxy)benzamide according to formula (1) 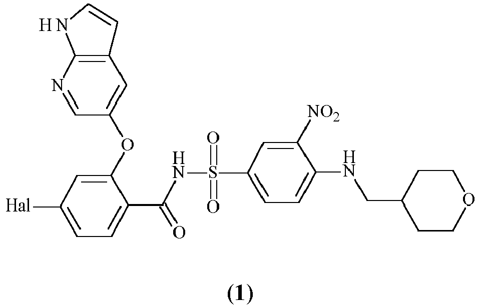 wherein Hal represents -Cl or -Br;
(f)
(**f₁**) providing optionally mono-protected piperazine according to formula (2) 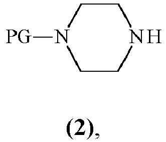 wherein PG represents -H or a protective group;
(**f₂**) providing 2-(4-chlorophenyl)-4,4-dimethyl-cyclohexene-1-carbaldehyde according to formula **(5)** 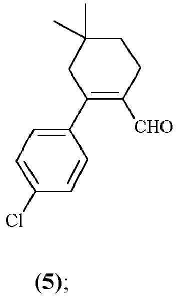
**(f₃)** reacting the 4-halo-N-[3-nitro-4-(tetrahydropyran-4-ylmethylamino)phenyl]sulfonyl-2-(1H-pyrrolo[2,3-b]pyridin-5-yloxy)benzamide according to formula **(1)** with the optionally mono-protected piperazine according to formula **(2)** thereby obtaining protected N-[3-nitro-4-(tetrahydropyran-4-ylmethylamino)phenyl]sulfonyl-4-piperazin-1-yl-2-(1H-pyrrolo[2,3-b]pyridin-5-yloxy)benzamide according to formula **(3)** 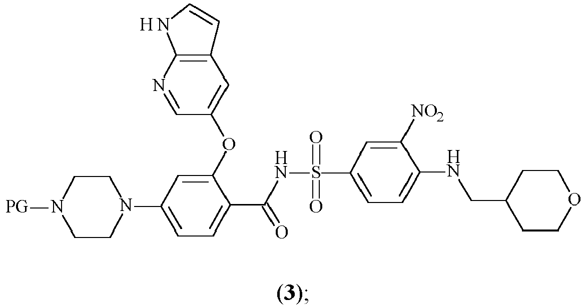
**(f₄)** optionally, when PG does not represent -H, cleaving the protective group PG from the protected N- [3 -nitro-4-(tetrahydropyran-4-ylmethylamino)phenyl] sulfonyl-4-piperazin-1-yl-2-(1H-pyrrolo[2,3-b]pyridin-5-yloxy)benzamide according to formula **(3);** thereby obtaining N-[3-nitro-4-(tetrahydropyran-4-ylmethylamino)phenyl]sulfonyl-4-piperazin-1-yl-2-(1H-pyrrolo[2,3-b]pyridin-5-yloxy)benzamide according to formula **(4)** 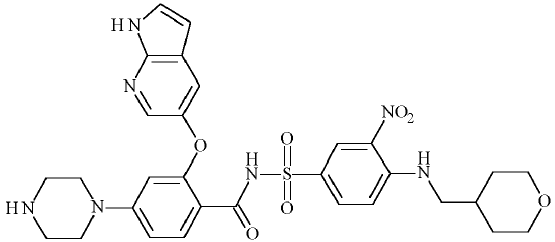 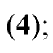 and
**(f₅)** reacting the N-[3-nitro-4-(tetrahydropyran-4-ylmethylamino)phenyl]sulfonyl-4-piperazin-1-yl-2-(1H-pyrrolo[2,3-b]pyridin-5-yloxy)benzamide **(4)** with the 2-(4-chlorophenyl)-4,4-dimethyl-cyclohexene-1-carbaldehyde according to formula **(5)** in the presence of a reducing agent;
or
**(f')**
**(f₁')** providing 1-[[2-(4-chlorophenyl)-4,4-dimethyl-cyclohexen-1-yl]methyl]piperazine according to formula **(6)** 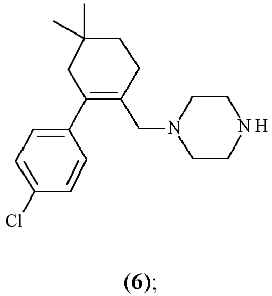
**(f₂')** reacting the 4-halo-N-[3-nitro-4-(tetrahydropyran-4-ylmethylamino)phenyl]sulfonyl-2-(1H-pyrrolo[2,3-b]pyridin-5-yloxy)benzamide according to formula (1) with the 1-[[2-(4-chlorophenyl)-4,4-dimethyl-cyclohexen-1-yl]methyl]piperazine according to formula **(6);**
thereby obtaining Venetoclax (VNT).

5. The process according to claim 4, wherein in step (**f₁**) and step **(f₄)** the protective group is tert-butyloxycarbonyl (Boc).

6. The process according to claim 4 or 5, wherein step **(f₃)** and step **(f₂')** is carried out in the presence of a base and in the presence of a catalyst; preferably a palladium catalyst.

7. The process according to any of claims 4 to 6, wherein in step (**f₅**) the reducing agent is NaBH(OAc)₃; preferably in toluene and THF.

8. The process according to any of claims 4 to 7, wherein step (**f₂**) comprises the steps
(fz-i) providing 3,3-dimethylcyclohexanone according to formula **(11)** 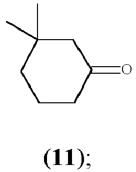
(**f₂-ii**) providing (4-chlorophenyl)boronic acid according to formula (13) 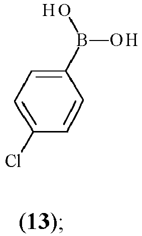
**(f₂-iii)** formylating the 3,3-dimethylcyclohexanone according to formula (11) with POCl₃ and dimethylformamide (DMF) thereby obtaining 2-chloro-4,4-dimethyl-cyclohexene-1-carbaldehyde according to formula (12) 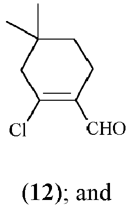
**(f₂-iv)** reacting the 2-chloro-4,4-dimethyl-cyclohexene-1-carbaldehyde according to formula (12) with the (4-chlorophenyl)boronic acid according to formula (13) thereby obtaining the 2-(4-chlorophenyl)-4,4-dimethyl-cyclohexene-1-carbaldehyde according to formula (5).

9. The process according to claim 8, wherein step **(f₂-iii)** is carried out in toluene; preferably at a temperature within the range of 50 to 55°C.

10. The process according to claim 8 or 9, wherein step **(f₂-iv)** is carried out in the presence of a base; preferably K₂CO₃; and in the presence of a catalyst; preferably Pd(PPh₃)₄; preferably in MeCN and H₂O; preferably at a temperature of 75 to 80°C.

11. The process according to any of claims 4 to 10, wherein step (**f₁'**) comprises the steps
(**f₁'-i**) providing 2-(4-chlorophenyl)-4,4-dimethyl-cyclohexene-1-carbaldehyde according to formula **(5)** 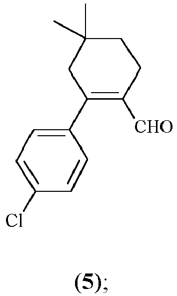
**(f₁'-ii)** providing optionally mono-protected piperazine according to formula **(2)** 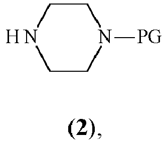 wherein PG represents -H or a protective group;
(**f₁'-iii**) reacting the 2-(4-chlorophenyl)-4,4-dimethyl-cyclohexene-1-carbaldehyde according to formula **(5)** with the optionally mono-protected piperazine according to formula (2) in the presence of a reducing agent thereby obtaining protected 1-[[2-(4-chlorophenyl)-4,4-dimethyl-cyclohexen-1-yl]methyl]piperazine according to formula **(14)** 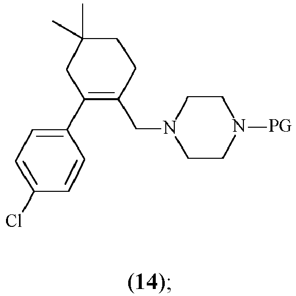
(**f₁'-iv**) optionally, when PG does not represent -H, cleaving the protective group from the protected 1-[[2-(4-chlorophenyl)-4,4-dimethyl-cyclohexen-1-yl]methyl]piperazine according to formula **(14);** thereby obtaining 1-[[2-(4-chlorophenyl)-4,4-dimethyl-cyclohexen-1-yl]methyl]piperazine dihydrochloride according to formula **(15)** 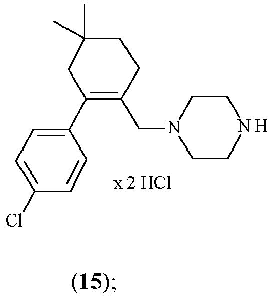 and
(**f₁'-v**) treating the 1-[[2-(4-chlorophenyl)-4,4-dimethyl-cyclohexen-1-yl]methyl]piperazine dihydrochloride according to formula **(15)** with a base thereby obtaining the 1-[[2-(4-chlorophenyl)-4,4-dimethyl-cyclohexen-1-yl]methyl]piperazine according to formula (6).

12. The process according to claim 11, wherein in step (**f₁'-ii**) and in step **(f₁'-iv)** the protective group is tert-butyloxycarbonyl (Boc); preferably wherein step **(f₁'-iv)** is carried out by adding HCl; preferably in solution of isopropanol; preferably at a temperature within the range of from 75 to 80°C.

13. The process according to claim 11 or 12, wherein in step (**f₁'-iii**) the reducing agent is NaBH(OAc)₃; preferably in toluene and THF.

14. 4-Halo-N-[3-nitro-4-(tetrahydropyran-4-ylmethylamino)phenyl]sulfonyl-2-(1H-pyrrolo[2,3-b]pyridin-5-yloxy)benzamide according to formula (1) 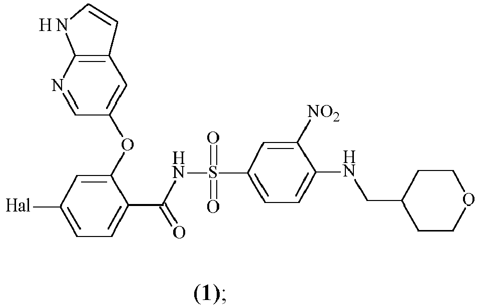 wherein Hal represents -Cl or -Br.

15. 4-Halo-2-fluoro-N-[3-nitro-4-(tetrahydropyran-4-ylmethylamino)phenyl] sulfonyl-benzamide according to formula (9) 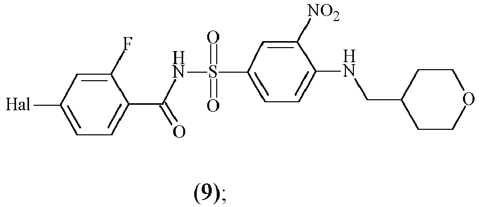 wherein Hal represents -Cl or -Br.
